(19) Europäisches Patentamt
European Patent Office
Office européen des brevets

(11) Veröffentlichungsnummer: **0 454 624 B1**

(12) # EUROPÄISCHE PATENTSCHRIFT

(45) Veröffentlichungstag der Patentschrift: **11.10.95**

(51) Int. Cl.$^6$: **C07C 45/72**, C07C 45/45, C07C 49/76

(21) Anmeldenummer: **91810290.6**

(22) Anmeldetag: **17.04.91**

(54) **Verfahren zur Herstellung von 1,3-Diketonen.**

(30) Priorität: **26.04.90 CH 1425/90**

(43) Veröffentlichungstag der Anmeldung:
**30.10.91 Patentblatt 91/44**

(45) Bekanntmachung des Hinweises auf die Patenterteilung:
**11.10.95 Patentblatt 95/41**

(84) Benannte Vertragsstaaten:
**BE DE FR GB IT**

(56) Entgegenhaltungen:
**DE-A- 2 439 282**
**DE-A- 3 302 123**
**FR-A- 1 202 737**
**US-A- 3 004 932**
**US-A- 4 387 089**

(73) Patentinhaber: **CIBA-GEIGY AG**
**Klybeckstrasse 141**
**CH-4002 Basel (CH)**

(72) Erfinder: **Drewes, Rolf, Dr.**
**Am Buchacker 1**
**W-6145 Lindenfels (DE)**
Erfinder: **Friedrich, Hans-Helmut**
**Am Rauenstein 8**
**W-6147 Lautertal 2 (DE)**
Erfinder: **Mehner, Hans-Ludwig**
**Guldenweg 9**
**W-6840 Lampertheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft ein Verfahren zur Herstellung von 1,3-Diketonen durch Claisen-Kondensation von Ketonen mit Estern in Gegenwart von Alkali- und Erdalkalimetallalkoholaten in organischen Lösungsmitteln oder Lösungsmittelgemischen, mit Ausnahme von Dimethylsulfoxid und Alkoholen, in denen die Base zu mindestens 8 % löslich ist.

1,3-Diketone sind als wertvolle Costabilisatoren für chlorhaltige Polymere, insbesondere Polyvinylchlorid, die gegen schädigenden Einfluss von Warme und/oder Licht geschützt werden müssen, aus der Literatur bekannt. Ausserdem sind 1,3-Diketone wichtige Ausgangsstoffe und Zwischenprodukte zur Synthese von Heterocyclen. Die Claisen-Kondensation ist als Herstellungsmethode für 1,3-Diketone allgemein bekannt und in zahlreichen Lehrbüchern der organischen Chemie, wie z.B. Organikum, VEB Deutscher Verlag der Wissenschaften, Berlin (1969), S. 580, 632, 658, J.B. Hendrickson, D.J. Cram, G.S. Hammond, Organic Chemistry, McGraw-Hill S. 522, 524, 525 oder J. March, Advanced Organic Chemistry, J. Wiley & Sons (1985), S. 437-39, 835 beschrieben.

Normalerweise wird die Reaktion zur Herstellung der Diketone in einem inerten organischen Lösungsmittel in Gegenwart von Alkalimetallen, Alkalimetallalkoholaten oder Alkalimetallhydriden als Base durchgeführt. So ist in der US-A 3,004,932 die Herstellung von ungesättigten $\beta$-Diketonen mit Alkalimetallalkoholaten in Diethylether veröffentlicht. Um die Diketone in reiner Form zu erhalten, wird dort eine Methode der Isolierung über die Ferri-Komplexe beschrieben.

In der FR-A 1 202 737 wird die Durchführung der Claisen-Kondensation in Alkoholen als Lösungsmittel mit in Wasser schwerlöslichen Alkalimetallalkoholat-Basen, die mindestens 5 Kohlenstoffatome enthalten, beschrieben.

In der US-A 4 387 089 wird die Herstellung von 4-(1,1-Dimethylethyl)-4'-methoxydibenzoylmethan mittels Claisen-Kondensation beschrieben.

In der DE-A 33 02 123 werden Claisen-Kondensationen mit Alkalimetallhydriden und -amiden durchgeführt.

Die DE-A 24 39 282 offenbart solche Reaktionen mit Natriumhydrid als Base.

Da in den bekannten Verfahren der Claisen-Kondensation hohe Ueberschüsse an Keton eingesetzt werden müssen und lange Reaktionszeiten erforderlich sind, besteht ein Interesse an verbesserten Verfahren zur Durchführung dieser Reaktion. Ausserdem ist die bisher angewandte übliche Methode der Isolierung und Reinigung der synthetisierten Diketone aufwendig. So ist es für die industrielle Anwendung von Vorteil, ein vereinfachtes Verfahren einzuführen.

Es wurde nun übertaschenderweise gefunden, dass durch die Verwendung von organischen inerten Lösungsmitteln oder Lösungsmittelgemischen, in denen die zugesetzten Basen zu mindestens 8 % löslich sind, das Verfahren besonders vorteilhaft durchzuführen ist. Es kann z.B. die Reaktionszeit verkürzt werden. Es werden hohe Ausbeuten und Reinheiten erzielt.

Gegenstand der Erfindung ist ein Verfahren zur Herstellung von linearen 1,3-Diketonen der allgmeinen Formel I

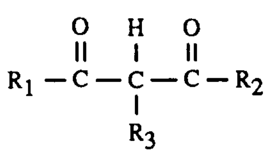

(I)

worin

$R_1$ und $R_2$ unabhängig voneinander für $C_1$-$C_{20}$-Alkyl, Phenyl, durch Halogen, Hydroxy, $NO_2$, $C_1$-$C_4$-Alkyl und/oder $C_1$-$C_4$-Alkoxy substituiertes Phenyl, $C_7$-$C_9$-Phenylalkyl oder für einen Rest der Formel II stehen

$-A-X-R_4$ (II)

wobei

A $C_1$-$C_{12}$-Alkylen, Phenylen, durch Halogen, Hydroxy, $NO_2$, $C_1$-$C_4$-Alkyl und/oder $C_1$-$C_4$-Alkoxy substituiertes Phenylen oder durch Hydroxy, Halogen oder/und Alkoxy substituiertes $C_1$-$C_{12}$-Alkylen bedeutet,

X für Sauerstoff oder Schwefel steht und

$R_4$ Wasserstoff, $C_1$-$C_{18}$-Alkyl, Phenyl, durch Halogen, Hydroxy, $C_1$-$C_4$-Alkyl, $NO_2$ und/oder $C_1$-$C_4$-Alkoxy substituiertes Phenyl oder $C_7$-$C_9$-Phenylalkyl bedeutet und $R_3$ Wasserstoff, $C_1$-$C_{20}$-Alkyl, Phenyl, durch

Halogen, Hydroxy, $C_1$-$C_4$-Alkyl, $NO_2$ und/oder $C_1$-$C_4$-Alkoxy substituiertes Phenyl oder $C_7$-$C_9$-Phenylalkyl darstellt, durch Claisen-Kondensation von Ketonen der Formel III

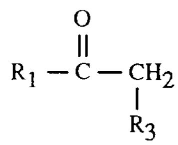

(III)

mit Estern der Formel IV

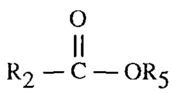

(IV)

worin $R_5$ für $C_1$-$C_5$-Alkyl, Phenyl, oder durch Halogen, $C_1$-$C_4$-Alkyl oder Hydroxy substituiertes Phenyl steht;
oder, wenn $R_2$ in Formel I $-(CH_2)_mOH$ bedeutet, auch mit cyclischen Estern der Formel V

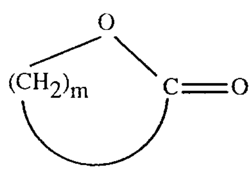

(V)

in der m 2 bis 10 bedeutet,
in Gegenwart von Natrium-n-butylat, Natrium-tert-butylat oder Natriumamylat als Base, dadurch gekennzeichnet, dass man die Umsetzung in einem inerten organischen Lösungsmittel oder Lösungsmittelgemisch aus der Gruppe der cyclischen oder linearen Ether oder cyclischen oder linearen Amide, in denen die Base zu mindestens 8 % löslich ist, durchführt, wobei das Lösungsmittel(gemisch) kein Dimethylsulfoxid ist (enthält) und das Lösungsmittel nicht aus einem Alkohol besteht.

$R_1$ und $R_2$ als $C_1$-$C_{20}$-Alkyl können linear oder verzweigt sein und beispielsweise Methyl, Ethyl, n-Propyl, Isopropyl, n-Butyl, tert-Butyl, n-Pentyl, Isopentyl, n-Hexyl, n-Heptyl, n-Octyl, Isooctyl, n-Nonyl, Decyl, Undecyl, Dodecyl, Tridecyl, Tetradecyl, Pentadecyl, Hexadecyl, Heptadecyl, Octadecyl, Nonadecyl oder Eicosyl, bevorzugt $C_1$-$C_{18}$-Alkyl, insbesondere z.B. Methyl, Isopentyl, n-Nonyl, Pentadecyl oder Heptadecyl bedeuten.

Ist $R_1$ oder $R_2$ substituiertes Phenyl, enthält letzteres z.B. 1 bis 3, insbesondere 1 oder 2 Substituenten.

$R_1$ und $R_2$ als ($C_1$-$C_4$-Alkyl)-phenyl können beispielsweise für mit 1-3, insbesondere 1 oder 2 Alkylgruppen, vor allem mit Methylgruppen substituiertes Phenyl stehen. Beispiele dafür sind Tolyl, Xylyl oder Mesityl.

$R_1$ und $R_2$ als durch Halogen substituiertes Phenyl können beispielsweise einen ein- oder mehrfach durch Fluor, Chlor, Brom oder Iod, insbesondere Chlor oder Brom substituierten Phenylring bedeuten, wie z.B. Chlorphenyl oder Dichlorphenyl.

$C_1$-$C_4$-Alkoxy bedeutet beispielsweise Methoxy, Ethoxy, Propyloxy oder Butoxy, ein entsprechend substituiertes Phenyl ist beispielsweise Methoxyphenyl.

$R_1$ und $R_2$ als $C_7$-$C_9$-Phenylalkyl stehen z.B. für Benzyl, Phenylethyl, $\alpha$-Methylbenzyl, 3-Phenylpropyl oder $\alpha,\alpha$-Dimethylbenzyl, bevorzugt ist Benzyl.

Bevorzugt sind $R_1$ und $R_2$ $C_1$-$C_{18}$-Alkyl, Phenyl, ($C_1$-$C_4$-Alkyl)phenyl oder -A-X-$R_4$.

A als $C_1$-$C_{12}$-Alkylen kann beispielsweise ein lineares oder verzweigtes, vorzugsweise lineares, Alkylen bedeuten. Beispiele für solche Reste ergeben sich aus den obigen Beispielen für Alkyl als $R_1$ und $R_2$ bis zur entsprechenden Anzahl der C-Atome durch Anfügen des Suffixes -en. Bevorzugt ist $C_1$-$C_6$-Alkylen, insbesondere n-Propylen oder n-Pentylen.

$R_4$ als $C_1$-$C_{18}$-Alkyl kann z.B. für lineares oder verzweigtes Alkyl stehen wie beispielsweise für $R_1$ und $R_2$ beschrieben bis zur entsprechenden Anzahl der C-Atome.

$R_4$ als substituiertes Phenyl oder $C_7$-$C_9$-Phenylalkyl kann die gleichen Bedeutungsmöglichkeiten haben wie für $R_1$ und $R_2$ beschrieben.

$R_4$ ist bevorzugt Wasserstoff, $C_1$-$C_{18}$-Alkyl oder Phenyl.

A als gegebenenfalls substituiertes Phenylen ist vorzugsweise o- oder p-Phenylen, insbesondere unsubstituiertes Phenylen.

$R_3$ als $C_1$-$C_{20}$-Alkyl, substituiertes Phenyl oder $C_7$-$C_9$-Phenylalkyl kann die gleichen Bedeutungsmöglichkeiten haben wie für $R_1$ und $R_2$ beschrieben, bevorzugt ist $C_1$-$C_4$-Alkyl.

$R_3$ steht bevorzugt für Wasserstoff oder $C_1$-$C_4$-Alkyl, ganz besonders bevorzugt ist $R_3$ Wasserstoff.

$R_5$ als $C_1$-$C_5$-Alkyl bedeutet beispielsweise Methyl, Ethyl, n-Propyl, Isopropyl, n-Butyl, tert-Butyl, n-Pentyl oder Isopentyl, bevorzugt ist Methyl.

$R_5$ als ($C_1$-$C_4$-Alkyl)-phenyl kann die gleichen Bedeutungsmöglichkeiten haben wie für $R_1$ und $R_2$ beschrieben.

Als unter den Reaktionsbedingungen inerte organische Lösungsmittel kommen beispielsweise lineare oder cyclische Ether oder cyclische oder lineare Amide in Frage.

Lineare oder cyclische Ether können beispielsweise Mono-, Di- Tri- oder Polyether sein. Beispiele dafür sind: Diethylether, Diisopropylether, Methyl-tert-butylether, Dibutylether, Ethylenglycoldimethylether, Diethylenglycoldimethylether, Tetrahydrofuran, Tetrahydropyran, Dioxan oder Dioxolan. Bevorzugt sind cyclische Ether und höhere lineare Ether (z.B. ab 5 C-Atomen), insbesondere Dioxan, Tetrahydrofuran oder Methyl-tert-butylether. Es können jeweils auch Mischungen dieser Lösungsmittel verwendet werden.

Als cyclisches oder lineares Amid kommt z.B. N-Methyl-Pyrrolidon in Frage.

Erfindungswesentlich ist, dass das verwendete Lösungsmittel die Base unter den Reaktionsbedingungen zu mindestens 8 Gew.% löst. Die Auswahl von brauchbaren Lösungsmitteln hängt somit von der verwendeten Base (unterschiedliche Löslichkeiten) sowie von der Reaktionstemperatur ab. Somit können einige der oben beispielhaft aufgezählten Lösungsmittel für bestimmte Basen oder/und Reaktionstemperaturen brauchbar sein, für andere hingegen nicht.

Das Reaktionsgemisch kann auch geringe Anteile des zum jeweils verwendeten Alkalialkoholat korrespondierenden Alkohols enthalten, beispielsweise weniger als 5 %, insbesondere weniger als 1 %. Besonders zweckmässig enthält das Reaktionsgemisch keinen Alkohol.

Von besonderen, Interesse ist die Herstellung von Verbindungen, worin
$R_1$ und $R_2$ unabhängig voneinander $C_1$-$C_{20}$-Alkyl, Phenyl, ($C_1$-$C_4$-Alkyl)-phenyl oder einen Rest der Formel II bedeuten,
A für $C_1$-$C_6$-Alkylen steht,
$R_4$ Wasserstoff, $C_1$-$C_{18}$-Alkyl, Phenyl oder ($C_1$-$C_4$-Alkyl)-phenyl darstellt und
$R_3$ für Wasserstoff und $C_1$-$C_4$-Alkyl steht.

Bevorzugt werden Verbindungen der Formel I hergestellt, worin
$R_1$ und $R_2$ unabhängig voneinander $C_1$-$C_{18}$-Alkyl, Phenyl oder einen Rest der Formel II bedeuten,
$R_4$ Wasserstoff, Phenyl oder $C_1$-$C_{18}$-Alkyl darstellt und
$R_3$ für Wasserstoff steht.

Bevorzugt sind auch Verbindungen der Formel I, worin
$R_1$ und $R_2$ Phenyl oder einen Rest der Formel II bedeuten,
$R_4$ Phenyl und
X O bedeuten.

Besonders bevorzugt ist ein Verfahren, worin die organischen inerten Lösungsmittel cyclische oder lineare Ether sind.

Ganz besonders bevorzugt ist ein Verfahren, worin die inerten organischen Lösungsmittel cyclische Ether (insbesondere Dioxan und Tetrahydrofuran) oder Methyl-tert-butylether sind.

Zweckmässig ist die Durchführung des erfindungsgemässen Verfahrens bei Temperaturen zwischen -20 und +70 °C. Bevorzugt liegen die Reaktionstemperaturen z.B. zwischen -5 und +40 °C.

Die Reaktionszeiten des oben beschriebenen Verfahrens zur Claisen-Kondensation können in weiten Grenzen schwanken, liegen im allgemeinen aber zwischen 0,5 und 5,0 Stunden.

Als Säure zur Hydrolyse kommen beispielsweise Ameisensäure, Essigsäure, Phosphorsäure, Salzsäure oder Schwefelsäure in Frage, bevorzugt sind Salzsäure und Schwefelsäure.

Wie bereits eingangs erwähnt, stellen die erfindungsgemäss herstellbaren linearen 1,3-Diketone wertvolle Costabilisatoren für chlorhaltige Polymerisate, die gegen schädigenden Einfluss von Wärme und/oder Licht geschützt werden müssen, sowie wertvolle Ausgangsstoffe und Zwischenprodukte zur Synthese von Heterocyclen, dar. Es besteht daher ein Interesse daran, diese Diketone in möglichst einfachen Verfahren mit wenig Energieaufwand in hohen Ausbeuten herzustellen.

Das erfindungsgemässe Verfahren eröffnet einen technisch besonders günstigen und wirtschaftlichen Weg zu deren Herstellung.

Erfindungswesentlich ist, dass im oben beschriebenen Verfahren ein inertes organisches Lösungsmittel oder Lösungsmittelgemisch verwendet wird, in dem die eingesetzte Base unter den Reaktionsbedingungen zu mindestens 8 % löslich ist. Bevorzugt ist eine Löslichkeit von mindestens 10 %, vorzugsweise mindestens 15 %, insbesondere mindestens 20 %.

Ein wichtiger Vorteil des erfindungsgemässen Verfahrens liegt darin, dass zu seiner Durchführung relativ niedrige Reaktionstemperaturen erforderlich sind, ein wichtiger Aspekt der Energieersparnis für die industrielle Anwendung. So kann das Verfahren z.B. bei -20 bis 70 °C, vorzugsweise bei -5° bis +40 °C durchgeführt werden. In den bisher bekannten Verfahren ist die Verwendung eines bis zu 100%igen Ueberschusses an Keton zur Erlangung guter Ausbeuten erforderlich. Als besonderer technischer Vorteil des erfindungsgemässen Verfahrens ist daher auch die mögliche Erniedrigung des Edukt- und Basen-Ueberschusses im Vergleich zu den bisher bekannten Verfahren zu nennen. So ist es möglich, mit dem erfindungsgemässen Verfahren auch mit annähernd stöchiometrischen Mengen oder geringen Ueberschüssen an Ester hohe Ausbeuten zu erzielen.

Die benötigte Base und die Ester-Komponente setzt man zweckmässig in einer Menge von 0,5-1,5 Mol, bevorzugt von 0,65-1,25 Mol, insbesondere von 0,9-1,2 Mol, bezogen auf 1 Mol Keton ein.

Die Reaktion wird in an sich bekannter Weise durchgeführt, indem man z.B. die Base im Lösungsmittel vorlegt und die Ester- und Ketonkomponente nacheinander oder gleichzeitig zugibt. Uebliche Operationen wie Rühren des Reaktionsgemisches sind von Vorteil.

Wie dem Fachmann bekannt, können die Verbindungen der Formel I selbstverständlich teilweise oder ganz in den tautomeren Formen vorliegen nach dem Gleichgewicht:

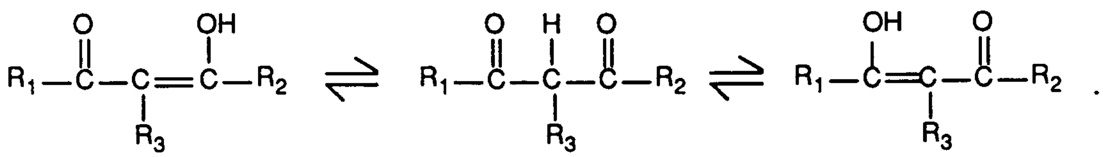

Die nachfolgenden Beispiele erläutern das erfindungsgemässe Verfahren weiter. Darin sowie in der übrigen Beschreibung und den Patentansprüchen bedeuten Teile Gewichtsteile und Prozentangaben Gewichtsprozent, sofern nichts anderes angegeben ist.

Beispiel 1:

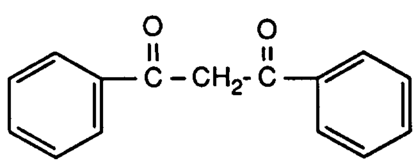

In einem 0,5 l Sovirel-Reaktor (doppelwandiges Reaktionsgefäss) mit Rührer, Thermometer und Tropftrichter werden 31,7 g Natrium-tert-butylat und 200 g Methyl-tert-butylether vorgelegt und auf 0 °C abgekühlt. Anschliessend werden 45 g Benzoesäuremethylester und 36 g Acetophenon als Gemisch über einen Zeitraum von 35 Minuten eingetropft. Das Reaktionsgemisch wird dann 45 Minuten bei 30 °C gerührt, das Lösungsmittel am Rotationsverdampfer bei einer Badtemperatur von < 60 °C abdestilliert und der Rückstand mit 800 ml Wasser gelöst. Anschliessend wird mit verdünnter Salzsäure angesäuert, der Niederschlag abfiltriert, mit Wasser gewaschen und getrocknet.
Ausbeute: 62,1 g ≙ 92,3 % der Theorie.
Schmelzpunkt: 72-75 °C.

Beispiel 2:

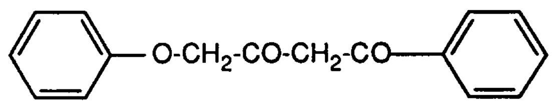

In eine Apparatur wie in Beispiel 1 beschrieben werden 31,7 g Natrium-tert-butylat und 200 g wasserfreies Tetrahydrofuran vorgelegt, auf 15 °C abgekühlt und innerhalb 50 Minuten ein Gemisch aus 54,8 g Phenoxyessigsäuremethylester und 36 g Acetophenon bei 15 °C unter Rühren eingetropft. Anschliessend wird das Gemisch jeweils 30 Minuten bei 15 °C und 30 °C nachgerührt. Dann werden die flüchtigen Anteile im Vakuum mittels Rotationsverdampfer abdestilliert, der Rückstand mit 500 ml Eiswasser aufgenommen, mit 60 g 25%iger Schwefelsäure angesäuert und der ausgefallene Festkörper abgesaugt, mit Wasser gewaschen und getrocknet.
Ausbeute: 73,6 g ≙ 96,5 % der Theorie, gelbe Kristalle mit einem Schmelzpunkt von 72-74 °C.

Nach dem Umkristallisieren aus Isopropanol/Wasser erhält man ein Produkt mit dem Schmelzpunkt 81-82 °C.

Beispiel 3:

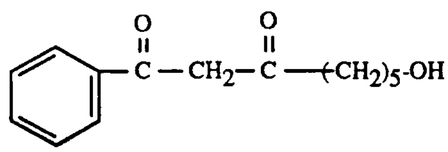

In einem 0,5-l-Sovirel-Kolben (doppelwandiges Reaktionsgefäss) mit Rührer, Thermometer, Tropftrichter und Destillationsvorlage werden 52,9 g Natrium-tert.-butylat (0,55 mol) und 300,0 g Tetrahydrofuran (THF) (Wassergehalt < 0,01 %) vorgelegt und auf 0 °C gekühlt. Bei dieser Temperatur wird eine Lösung von 60,0 g Acetophenon (99 %, 0,50 mol), 57,7 g ε-Caprolacton (99 %, 0,50 mol) und 30,0 g THF unter Rühren im Verlauf einer Stunde zudosiert. Dann wird 30 Minuten bei 0 °C und anschliessend 2 Stunden bei 20 °C nachgerührt. Dann werden bei 45 °C und erniedrigtem Druck (30 mbar) ca. 330 g Lösungsmittel abdestilliert.

Der Rückstand wird auf 30 °C gekühlt und mit 300 g Wasser versetzt. Die Lösung wird 3 mal mit je 70 ml Xylol extrahiert. Dann wird die wässrige Phase auf 1200 ml verdünnt und mit Salzsäure auf pH ~ 5-6 eingestellt. Das Produkt fällt aus, wird abfiltriert, mit 100 ml Wasser gewaschen und getrocknet.

Ausbeute: 76,3 g ≙ 65,8 % der Theorie, gelbliche Kristalle mit einem Schmelzpunkt von 46-49 °C.

Beispiel 4:

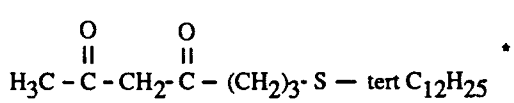

* -tert$C_{12}H_{25}$ = isomeres Gemisch mit der Hauptkomponente
$-C(CH_3)_2-CH_2-C(CH_3)_2-CH_2-C(CH_3)_3$

In einem 0,5 l-Sovirel-Kolben (doppelwandiges Reaktionsgefäss) mit Rührer, Thermometer, Tropftrichter und Kühler werden 37 g Natrium-tert-amylat in 200 g Dioxan suspendiert. Anschliessend wird ein Gemisch aus 11,6 g Aceton und 60,4 g 4-(tert-Dodecylmercapto)-buttersäuremethylester innerhalb von 25 Minuten bei 15 °C zugetropft. Man rührt 15 Minuten bei 15 °C und jeweils 45 Minuten bei 20 und 30 °C. Das Lösungsmittel wird am Rotationsverdampfer eingeengt, der Rückstand in 500 ml Wasser aufgenommen,

angesäuert und anschliessend dreimal mit Ether extrahiert. Die gesammelten organischen Phasen werden mit wenig Wasser und $NaHCO_3$-Lösung gewaschen, über $NaSO_4$ getrocknet, eingeengt und der Rückstand destilliert.
$Kp_{(0,3\ bar)}$: 138-142 °C
Brechungsindex: $n_D(20) = 1,4904$
Ausbeute: 58,4 g (90 % d. Theorie).

Beispiele 5-10:

Die in der folgenden Tabelle 1 angegebenen Verbindungen werden analog zur Vorschrift des Beispiels 2 hergestellt.

Tabelle 1:

| Beispiel Nr. | Verbindung | Lösungs-mittel | Base | Ausbeute [%] | Reinigung | Analytische Daten Schmelzpunkt/Siedepunkt Brechungsindex |
|---|---|---|---|---|---|---|
| 5 | 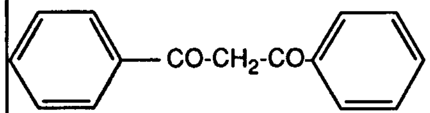 | Tetra-hydro-furan | $^{t}C_5H_{11}$-ONa | 82,7 | A | 76 - 77 °C |
| 6 | 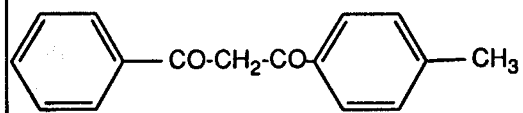 | Dioxan | $^{t}C_4H_9$-ONa | 67,2 | A | 70 - 76 °C |
| 7 | 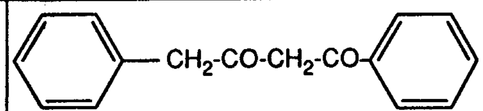 | Dioxan | $^{t}C_5H_{11}$-ONa | 87,0 | A | 47 - 48 °C |
| 8 | 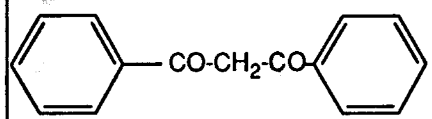 | Dioxan | $^{i}C_3H_7$-ONa | 55,0 laut Gas-chromato-graphie | - | - |
| 9 | 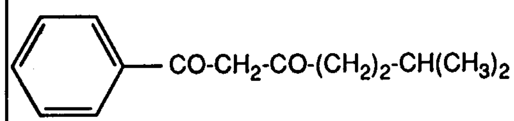 | Dioxan | $^{t}C_4H_9$-ONa | 75,0 | B | $Kp_{0,3}$: 98 - 103 °C<br>$n_D^{20}$: 1,5546 |
| 10 | 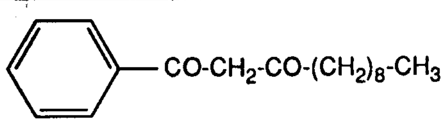 | Tetra-hydro-furan | $^{t}C_4H_9$-ONa | 75,0 | B | $Kp_{0,13}$: 139 - 141 °C<br>Smp.: 37 - 39 °C |

A Umkristallisation aus i-Propanol
B Destillation

Beispiel 11:

Die in Beispiel 1 beschriebene Herstellung von Dibenzoylmethan wird mit verschiedenen Lösungsmitteln durchgeführt und die Löslichkeit der Base im Lösungsmittel, sowie die Ausbeute an Dibenzoylmethan werden bestimmt.

Die Ergebnisse sind in Tabelle 2 aufgelistet.

Tabelle 2

| Beispiel | Lösungsmittel (200 g) | Temperatur in °C | Löslichkeit von Natrium-tert-butylat in % | Ausbeute in % |
|---|---|---|---|---|
| 5a | Methyl-tert-butylether | 0 | 27,3 | 92,3 |
| 5b | Tetrahydrofuran | 0 | 28,1 | 91,9 |
| 5c | Dioxan | 20 | 10,8 | 94,7 |

**Patentansprüche**

**1.** Verfahren zur Herstellung von linearen 1,3-Diketonen der allgmeinen Formel I

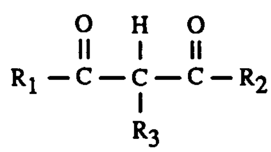
(I)

worin

$R_1$ und $R_2$ unabhängig voneinander für $C_1$-$C_{20}$-Alkyl, Phenyl, durch Halogen, Hydroxy, $NO_2$, $C_1$-$C_4$-Alkyl und/oder $C_1$-$C_4$-Alkoxy substituiertes Phenyl, $C_7$-$C_9$-Phenylalkyl oder für einen Rest der Formel II stehen

$-A-X-R_4$ (II)

wobei

A $C_1$-$C_{12}$-Alkylen, Phenylen, durch Halogen, Hydroxy, $NO_2$, $C_1$-$C_4$-Alkyl und/oder $C_1$-$C_4$-Alkoxy substituiertes Phenylen oder durch Hydroxy, Halogen oder/und Alkoxy substituiertes $C_1$-$C_{12}$-Alkylen bedeutet,

X für Sauerstoff oder Schwefel steht und

$R_4$ Wasserstoff, $C_1$-$C_{18}$-Alkyl, Phenyl, durch Halogen, Hydroxy, $C_1$-$C_4$-Alkyl, $NO_2$ und/oder $C_1$-$C_4$-Alkoxy substituiertes Phenyl oder $C_7$-$C_9$-Phenylalkyl bedeutet und

$R_3$ Wasserstoff, $C_1$-$C_{20}$-Alkyl, Phenyl, durch Halogen, Hydroxy, $C_1$-$C_4$-Alkyl, $NO_2$ und/oder $C_1$-$C_4$-Alkoxy substituiertes Phenyl oder $C_7$-$C_9$-Phenylalkyl darstellt, durch Claisen-Kondensation von Ketonen der Formel III

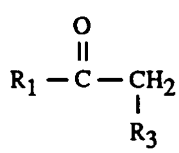
(III)

mit Estern der Formel IV

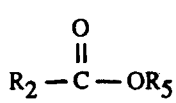
(IV)

worin $R_5$ für $C_1$-$C_5$-Alkyl, Phenyl, oder durch Halogen, $C_1$-$C_4$-Alkyl oder Hydroxy substituiertes Phenyl steht:

oder, wenn $R_2$ in Formel I $-(CH_2)_mOH$ bedeutet, auch mit cyclischen Estern der Formel V

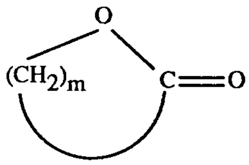

(V)

in der m 2 bis 10 bedeutet,
in Gegenwart von Natrium-n-butylat, Natrium-tert-butylat oder Natriumamylat als Base, dadurch gekennzeichnet, dass man die Umsetzung in einem inerten organischen Lösungsmittel oder Lösungsmittelgemisch aus der Gruppe der cyclischen oder linearen Ether oder cyclischen oder linearen Amide, in denen die Base zu mindestens 8 % löslich ist, durchführt, wobei das Lösungsmittel(gemisch) kein Dimethylsulfoxid ist (enthält) und das Lösungsmittel nicht aus einem Alkohol besteht.

2. Verfahren nach Anspruch 1, worin die Base in den verwendeten Lösungsmitteln oder Lösungsmittelgemischen zu mindestens 10 % löslich ist.

3. Verfahren nach Anspruch 1, worin die organischen inerten Lösungsmittel lineare oder cyclische Ether sind.

4. Verfahren nach Anspruch 3, worin die organischen inerten Lösungsmittel cyclische Ether oder Methyl-tert-butylether sind.

5. Verfahren nach Anspruch 1, worin
$R_1$ und $R_2$ unabhängig voneinander $C_1$-$C_{20}$-Alkyl, Phenyl, ($C_1$-$C_4$-Alkyl)-phenyl oder einen Rest der Formel II bedeuten,
A für $C_1$-$C_6$-Alkylen steht,
$R_4$ Wasserstoff, $C_1$-$C_{18}$-Alkyl, Phenyl oder ($C_1$-$C_4$-Alkyl)-phenyl darstellt und
$R_3$ für Wasserstoff und $C_1$-$C_4$-Alkyl steht.

6. Verfahren nach Anspruch 5, worin
$R_1$ und $R_2$ unabhängig voneinander $C_1$-$C_{18}$-Alkyl, Phenyl oder einen Rest der Formel II bedeuten,
$R_4$ Wasserstoff, Phenyl oder $C_1$-$C_{18}$-Alkyl darstellt und
$R_3$ für Wasserstoff steht.

7. Verfahren nach Anspruch 6, worin $R_1$ und $R_2$ Phenyl oder einen Rest der Formel II bedeuten, $R_4$ Phenyl und X O bedeuten.

8. Verfahren nach Anspruch 1, worin die Claisen-Kondensationsreaktion bei Temperaturen zwischen -20 und +70 °C durchgeführt wird.

## Claims

1. A process for the preparation of a linear 1,3-diketone of general formula I

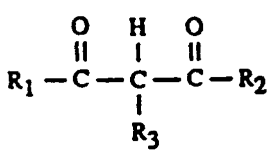

(I)

wherein
$R_1$ and $R_2$ are each independently of the other $C_1$-$C_{20}$alkyl, phenyl or phenyl which is substituted by halogen, hydroxyl, $NO_2$, $C_1$-$C_4$alkyl and/or $C_1$-$C_4$alkoxy, or are $C_7$-$C_9$phenylalkyl or a radical of formula II

$-A-X-R_4$ (II)

wherein
A is $C_1$-$C_{12}$alkylene, phenylene or phenylene which is substituted by halogen, hydroxyl, $NO_2$, $C_1$-$C_4$alkyl and/or $C_1$-$C_4$alkoxy, or is $C_1$-$C_{12}$alkylene which is substituted by hydroxyl, halogen and/or alkoxy,
X is oxygen or sulfur, and
$R_4$ is hydrogen, $C_1$-$C_{18}$alkyl, phenyl or phenyl which is substituted by halogen, hydroxyl, $C_1$-$C_4$alkyl, $NO_2$ and/or $C_1$-$C_4$alkoxy, or is $C_7$-$C_9$phenylalkyl, and
$R_3$ is hydrogen, $C_1$-$C_{20}$alkyl, phenyl or phenyl which is substituted by halogen, hydroxyl, $C_1$-$C_4$alkyl, $NO_2$ and/or $C_1$-$C_4$alkoxy, or is $C_7$-$C_9$phenylalkyl,
by Claisen condensation of a ketone of formula III

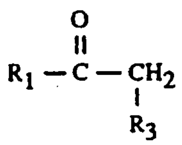

(III)

with an ester of formula IV

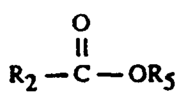

(IV)

wherein $R_5$ is $C_1$-$C_5$alkyl, phenyl or phenyl which is substituted by halogen, $C_1$-$C_4$alkyl or hydroxyl;
or, if $R_2$ in formula I is $-(CH_2)_mOH$, with a cyclic ester of formula V

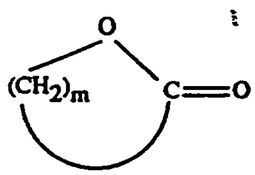

(V)

in which m is 2 to 10,
in the presence of sodium n-butylate, sodium tert-butylate or sodium amylate as base, which process comprises carrying out the reaction in an inert organic solvent or solvent mixture from the group consisting of cyclic and linear ethers and linear and cyclic amides in which the solubility of the base is at least 8%, which solvent may not be, or which solvent mixture may not contain, dimethyl sulfoxide, and which solvent is not an alcohol.

2. A process according to claim 1, wherein the solubility of the base in the solvent or solvent mixture used is at least 10%.

3. A process according to claim 1, wherein the inert organic solvent is a linear or cyclic ether.

4. A process according to claim 3, wherein the inert organic solvent is a cyclic ether or methyl tert-butyl ether.

5. A process according to claim 1, wherein $R_1$ and $R_2$ are each independently of the other $C_1$-$C_{20}$alkyl, phenyl, ($C_1$-$C_4$alkyl)phenyl or a radical of formula II,
A is $C_1$-$C_6$alkylene,
$R_4$ is hydrogen, $C_1$-$C_{18}$alkyl, phenyl or ($C_1$-$C_4$alkyl)phenyl, and
$R_3$ is hydrogen or $C_1$-$C_4$alkyl.

6. A process according to claim 5, wherein
$R_1$ and $R_2$ are each independently of the other $C_1$-$C_{18}$alkyl, phenyl or a radical of formula II,

$R_4$ is hydrogen, phenyl or $C_1$-$C_{18}$alkyl, and
$R_3$ is hydrogen.

**7.** A process according to claim 6, wherein $R_1$ and $R_2$ are phenyl or a radical of formula II, $R_4$ is phenyl and X is O.

**8.** A process according to claim 1, wherein the Claisen condensation reaction is carried out at a temperature between -20 and +70 °C.

**Revendications**

**1.** Procédé pour préparer des 1,3-dicétones linéaires de formule générale I

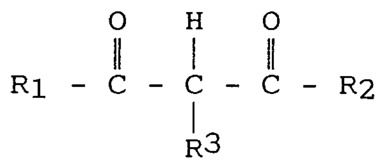

(I)

dans laquelle

$R_1$ et $R_2$, indépendamment l'un de l'autre, sont des radicaux alkyle en $C_1$-$C_{20}$, phényle, phényle substitué par des substituants halogéno, hydroxy, $NO_2$, alkyle en $C_1$-$C_4$ et/ou alcoxy en $C_1$-$C_4$, phénylalkyle en $C_7$-$C_9$, ou encore des radicaux de formule II

$$-A-X-R_4 \quad \text{(II)}$$

dans laquelle

A est un radical alkylène en $C_1$-$C_{12}$, phénylène, phénylène substitué par des substituants halogéno, hydroxy, $NO_2$, alkyle en $C_1$-$C_4$ et/ou alcoxy en $C_1$-$C_4$, ou alkylène en $C_1$-$C_{12}$ substitué par des substituants hydroxy, halogéno et/ou alcoxy,

X est un oxygène ou un soufre, et

$R_4$ est un hydrogène ou un radical alkyle en $C_1$-$C_{18}$, phényle, phényle substitué par des substituants halogéno, hydroxy, alkyle en $C_1$-$C_4$, $NO_2$ et/ou alcoxy en $C_1$-$C_4$, ou bien phénylalkyle en $C_7$-$C_9$, et

$R_3$ est un hydrogène ou un radical alkyle en $C_1$-$C_{20}$, phényle, phényle substitué par des substituants halogéno, hydroxy, alkyle en $C_1$-$C_4$, $NO_2$ et/ou alcoxy en $C_1$-$C_4$, ou bien phénylalkyle en $C_7$-$C_9$,

par une condensation de Claisen de cétones de formule III

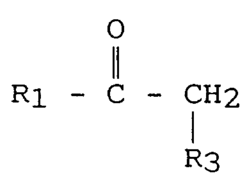

(III)

avec des esters de formule IV

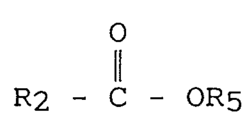

(IV)

dans laquelle $R_5$ est un radical alkyle en $C_1$-$C_5$, phényle, ou phényle substitué par des substituants halogéno, alkyle en $C_1$-$C_4$ ou hydroxy ;

ou bien, quand $R_2$, dans la formule I, est $-(CH_2)_mOH$, avec des esters cycliques de formule V

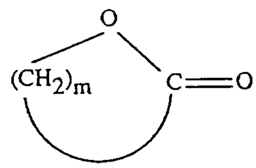

(V)

dans laquelle m vaut de 2 à 10,

en présence, comme base, de n-butylate de sodium, de tert-butylate de sodium ou d'amylate de sodium,

caractérisé en ce qu'on met en oeuvre la réaction dans un solvant ou dans un mélange de solvants organiques inertes choisis parmi l'ensemble comprenant les éthers cycliques ou linéaires ou les amides cycliques ou linéaires, dans lesquels la base est soluble à au moins 8 %, le solvant ou le mélange de solvants respectivement n'étant et ne contenant pas de diméthylsulfoxyde, le solvant n'étant pas constitué d'un alcool.

**2.** Procédé selon la revendication 1, dans lequel la base est soluble à au moins 10 % dans les solvants ou les mélanges de solvants utilisés.

**3.** Procédé selon la revendication 1, dans lequel les solvants organiques sont des éthers linéaires ou cycliques.

**4.** Procédé selon la revendication 3, dans lequel les solvants organiques inertes sont des éthers cycliques ou l'éther de méthyle et de tert-butyle.

**5.** Procédé selon la revendication 1, dans lequel

$R_1$ et $R_2$, indépendamment l'un de l'autre, sont des radicaux alkyle en $C_1$-$C_{20}$, phényle, (alkyle en $C_1$-$C_4$)-phényle, ou encore un radical de formule II,

A est un radical alkylène en $C_1$-$C_6$,

$R_4$ est un hydrogène ou un radical alkyle en $C_1$-$C_{18}$, phényle ou (alkyle en $C_1$-$C_4$)-phényle, et

$R_3$ est un hydrogène ou un radical alkyle en $C_1$-$C_4$.

**6.** Procédé selon la revendication 5, dans lequel

$R_1$ et $R_2$, indépendamment l'un de l'autre, sont des radicaux alkyle en $C_1$-$C_{18}$, phényle, ou encore un radical de formule II,

$R_4$ est un hydrogène, le radical phényle ou un radical alkyle en $C_1$-$C_{18}$, et

$R_3$ est un hydrogène.

**7.** Procédé selon la revendication 6, dans lequel $R_1$ et $R_2$ sont des radicaux phényle ou des radicaux de formule II, $R_4$ est le radical phényle et X et O.

**8.** Procédé selon la revendication 1, dans lequel la réaction de condensation de Claisen est mise en oeuvre à des températures comprises entre -20 et +70 °C.